# EUROPEAN PATENT SPECIFICATION

(11) **EP 2 403 822 B1**
(45) Date of publication and mention of the grant of the patent: **26.11.2014**
(21) Application number: 10711626.1
(22) Date of filing: 22.02.2010
(51) Int. Cl.: C07C 69/70, C12N 1/38

(54) **USE OF DATEM IN PRODUCTION MEDIA FOR LACTIC ACID BACTERIA**
VERWENDUNG VON DATEM IM KULTURMEDIUM FUER MILCHSÄURE-BAKTERIEN
UTILISATION DU DATEM DANS UN MILIEU DE CULTURE POUR BACTÉRIE D'ACIDE LACTIQUE

(30) Priority: 03.03.2009 EP 09154176
(43) Date of publication of application: 11.01.2012
(73) Proprietor: Chr. Hansen A/S, 2970 Hoersholm (DK)
(72) Inventor: KRINGELUM, Boerge, Windel, DK-2750 Ballerup (DK); SOERENSEN, Niels, Martin, DK-2450 Copenhagen SV (DK)
(86) International application number: PCT/EP2010/052193
(87) International publication number: WO 2010/100047

(56) References cited:
- US-A- 5 908 862
- US-A- 5 981 587
- US-A1- 2007 178 213
- US-B1- 6 642 278
- CORCORAN B M ET AL: "Growth of probiotic lactobacilli in the presence of oleic acid enhances subsequent survival in gastric juice" MICROBIOLOGY (READING), vol. 153, no. Part 1, January 2007 (2007-01), pages 291-299, XP002523924 ISSN: 1350-0872
- WITTENBERGER C L ET AL: "Tween 80 effect on glucosyltransferase synthesis by Streptococcus salivarius." JOURNAL OF BACTERIOLOGY JAN 1978, vol. 133, no. 1, January 1978 (1978-01), pages 231-239, XP002523925 ISSN: 0021-9193

## Description

### FIELD OF THE INVENTION

The present invention relates to the field of microbial cultures. More specifically, the invention provides a method for preparing a lactic acid bacteria (LAB) culture wherein the lactic acid bacteria is inoculated in a culture medium comprising comprises diacetyl tartaric acid esters of mono- and di-glycerides (DATEM). Such starter cultures are useful in the manufacturing of food, feed and pharmaceutical products.

### TECHNICAL BACKGROUND OF THE INVENTION

Microbial cultures are used extensively in the food, feed and pharmaceutical industry in the manufacturing of fermented products including most dairy products such as cheese, yoghurt and butter, but also in meat, bakery, wine or vegetable products. Furthermore, microbial cultures are also used to produce proteins including enzymes and various kinds of useful compounds. Such microbial cultures, which usually are upconcentrated, are referred to as starter cultures and are produced at industrial propagation plants and distributed to the fermentation industry, such as to a dairy plant, where the starter culture is used in their production processes.

In particularly cultures of lactic acid bacteria are widely used as starter cultures.

As used herein the term "lactic acid bacterium" (LAB) designates a gram-positive, microaerophilic or anaerobic bacterium which ferments sugars with the production of acids including lactic acid (as the predominantly produced acid), acetic acid and propionic acid. The industrially most useful lactic acid bacteria are found among Lactococcus species (spp.), Streptococcus spp., Lactobacillus spp., Leuconostoc spp., Pediococcus spp., Brevibacterium spp , Enterococcus spp. and Propionibacterium spp. Additionally, lactic acid producing bacteria belonging to the group of the strict anaerobic bacteria, bifidobacteria, i.e. Bifidobacterium spp. which are frequently used as food starter cultures alone or in combination with lactic acid bacteria, are generally included in the group of lactic acid bacteria. Even certain bacteria of the species Staphylococcus (e.g.: S. carnosus, S. equorum, S. sciuri, S. vitulinus and S. xylosus) have been referred to as LAB (Mogensen et al. (2002) Bulletin of the IDF No. 377, 10-19).

The publication of a large amount of reports documenting that various lactic bacteria beneficially affect the well-being of humans and/or animals has attracted even further interest to this group of bacteria. In particular, specific strains of *Lactobacillus* or *Bifidobacterium* have been found to be able to colonize the intestinal mucosa and to assist in the maintenance of the well-being of the hosts.

Probiotic microorganisms has been defined as "Live microorganisms which when administered in adequate amounts confer a health benefit on the host" (FAO / WHO 2002). During the recent years, documentation on probiotic properties of *Bifidobacteria* and other lactic bacteria has accumulated. In general the probiotic activity is associated with specific strains.

Both in the case of starter culture production and production of cultures for the preparation of probiotic compositions the yield during propagation is of central importance to the culture producers. The yield is often measured in number of living bacteria, culture forming units, CFU.

The production of LAB starter cultures involves the inoculation of LAB cells in a specific fermentation medium with an appropriate number of the cells to be propagated under appropriate fermentation conditions. In the industrial setting much effort is put into obtaining that the concentration of the propagated cells is high. This makes heavy demands on the fermentation conditions and the fermentation medium, which has to support growth of the cells in order to obtain the desired high yields.

To keep production costs low, industrial fermentations are normally carried out using complex, undefined fermentation media. Major components of such media can be yeast extract, cornstarch, whey protein or other milk-based media.

In addition to the major media components modern production media comprise a number of components which improve the yield of the production even when added at a ppm level.

One particular group of such components is constituted by a few emulgators.

The prime growth-promoting emulgator is polyoxyethylene-sorbitan-mono-oleate (Polysorbate 80, Tween 80®, CAS Registry Number: 9005-65-6). The addition of Tween 80 to the growth medium enhances the growth of many LAB, and accordingly Tween 80 is added to the majority of growth media of lactobacilli, including the standard MRS medium (Johnsson et al. APPLIED AND ENVIRONMENTAL MICROBIOLOGY, 61 (12): 4497. (1995); de Man et al. (1960). J. of Appl. Bact. 23 (I), 130-135.)).

Although Tween 80 in general possesses very low toxicity potential, health concerns have been raised with respect to it.

Tween 80 has been reported to have an effect on the central nerve system of experimental animals (Brubaker et al (1982) Life Sciences Vol 30, p 1965-71.), it has been associated with toxic effects to the liver, heart and kidney (Nityanand. S, Kapoor. NK (1979) "Effect. of chronic oral administration of Tween-80 in Charles Foster Rats". Indian. J Med. Res. 1979;69:664-670), and Tween 80 has been linked to fatalities among low birth weight (<1,500 g), premature infants in neonatal intensive care units (Alade (1986) PEDIATRICS Vol. 77, 593-597).

Furthermore, when culturing certain LAB e.g. Lactobacillus gasseri and strain Lactobacillus acidophilus strain LA-5 (DSM13241) present day Tween 80 comprising media are only providing low to moderate yields in the industrial setting.

Thus it would be highly advantageous to find a safe alternative to Tween 80 which provides similar high or even higher yields.

US2007/178213A1 discloses a yogurt comprising DATEM and lactic acid bacteria (Lactobacillus Bifidobacterium spp), which is packaged in a manner suitable for storage and handling.
In US2007/178213A1 is not described use of DATEM in a production process for preparing a lactic acid bacterial culture as such, wherein DATEM is used in the production bacterial growth medium as such and the cells are subsequently harvested/isolated.

### SUMMARY OF THE INVENTION

The above problem has been solved by substituting Tween 80 with the emulsifying diacetyltartaric acid esters of mono- and diglycerides also known as DATEMs.

It has surprisingly been found that DATEM can substitute Tween 80 in the growth media of Lactic Acid Bacteria. Actually, in the cultures of e.g. Lactobacillus gasseri and Lactobacillus acidophilus the use of DATEM result in higher cell numbers compared to Tween 80 based LAB growth media.

Thus one aspect of the invention relates to a process for preparing a lactic acid bacterial culture, comprising:
(a): culturing at least one strain of lactic acid bacteria in a bacterial growth medium, wherein the bacterial growth medium comprises at least 50 ppm of diacetyl tartaric acid esters of mono- and di-glycerides (DATEM); and
(b): harvesting the cultured bacteria.

DATEM is a commercially available well known compound for the skilled person.
A CAS registry number for a suitable DATEM is 91052-83-4.

This is indeed surprising because DATEM is reported to possess antibacterial activity (US05908862) and synergistically to increase the antibacterial effect of various substances (WO05104878A1).
US5981587 also describes antibacterial properties of DATEM whilst the present application shows the surprising effect of using the same product to support bacterial growth.

When producing bacterial cultures for human or animal intake the health issue is of paramount importance. In the USA, DATEM has been designated generally recognized as safe (GRAS) by the United States of America Food and Drug Administration (FDA) as specified in the Code of Federal Regulations, (21CFR184.1101 and CFR182.4101). DATEM is also not classified as dangerous according to EU-directive 91/155 (MSDS on PANODAN SD-K, Danisco Ingredients, USA, 05.05.99). It does not display toxicological effects in rats on 10% in diet corresponding to 5000 mg/kg body weight and is permitted generally in foodstuffs (Food additives in Europe 2000, Nordic Council of Ministers. Copenhagen 2002, ISBN 92-893-0829-X).

The fact that DATEM is recognized as safe is a big advantage in relation to e.g. use of a bacterial culture (e.g. as a yogurt culture) obtained by a process according to the invention.
As know to the skilled person - such a herein relevant bacterial culture may be used for the preparation of a food or feed, food- or feed supplements or for the preparation of pharmaceutical products or medicaments.

### DETAILED DISCLOSURE OF THE INVENTION

It is a primary result of the present invention that DATEM can substitute Tween 80 in the growth media of Lactic Acid Bacteria.

As demonstrated in example 1 and 2 it is possible to substitute Tween 80 with DATEM in a production medium while maintaining a high yield. Surprisingly it was found that the substitution of Tween 80 with DATEM resulted in even higher yields measured by colony forming units (CFU). Furthermore bacteria grown in DATEM are significantly less elongated (shorter). Elongated cells often result in formation of cell-lumps which may give separations problems in the down stream processing.

In general DATEM is comprised by diacetyl tartaric acid esters of mono- and diglycerides derived from food grade vegetable and/or animal triglycerides, may include glycerides comprising lauric, palmitic, stearic, oleic, linoleic and/or linolenic acids depending on the source of glycerides.

The membrane of LAB comprises a number of different fatty acids. Streptococcus thermophilus for instance has a high content around 50% of oleic acid, followed by palmitic acid and C20:1 fatty acids as the second most prevalent (15-20%). Thus unsaturated fatty acids are dominating.

According to Nieman (Microbiol. Mol. Biol. Rev. 18 (2): 147. (1954)) fatty acids of unsaturated nature are indispensable for some LAB, e.g. Lactobacillus helveticus, L. leichmannii, L. bulgarius, L. delbrackii (some strains), L. plantarum, S. cremonis and S. lactis (some strains), and may stimulate the growth of many others.

In general, vegetable oils comprise a higher amount of unsaturated fatty acids than oils from animal sources, therefore a bacterial growth medium, wherein the DATEM is a diacetyl tartaric acid ester of mono- and/or diglycerides made from vegetable oils is preferred. In particularly an embodiment wherein the DATEM is made from oil comprising unsaturated fatty-acid residues appear advantageous.

Interestingly, fatty acids may both inhibit or promote the growth of bacteria. Growth inhibition is affected by both saturated and unsaturated fatty acids. The antibacterial activity of unsaturated fatty acids increases with the number of double bonds in the molecule, and natural cis-forms are generally more active than the trans-isomers. Growth stimulating properties are exhibited mostly by unsaturated long-chain fatty acids (C18), (NIEMAN (1954) Bacteriol Rev.; 18(2): 147-163.).

The composition of sunflower oil and rapeseed oil has been reported to contain a substantial fraction of mono-unsaturated fatty acids (US07217875, Alonso (2006) Fuel Processing Technology 87, 97 - 102). Therefore a preferred embodiment of the invention is a medium, wherein the DATEM is a diacetyl tartaric acid ester of mono- and/or diglycerides made from sunflower and/or rapeseed oil.

Nieman (1954) also reports that certain strains of LAB display an absolute requirement for oleic acid (cis-9-octadecenoic acid). The membrane of most LAB comprises oleic acid E.g. the membrane of Streptococcus thermophilus has a high content around 50% of oleic acid.

Oleic acid is known to incorporate into the membranes of lactic acid bacteria (LAB) grown in a medium supplemented with Tween 80 (polyoxyethylene-sorbitan-mono-oleate). The addition of Tween 80 to the growth medium enhances the growth of many, if not all LAB, and Tween 80 is therefore often added to the growth media of lactobacilli, e.g., the standard MRS medium (Johnsson et al. (1995) APPLIED AND ENVIRONMENTAL MICROBIOLOGY, 61 (12): 4497).

While not wishing to be bound by any theory of operability, it is believed that Tween 80 represents an oleic acid reservoir. Tween 80 forms micelles that can not be imported by the cells. It is, however, contemplated that esterases and lipases released from the lactic acid bacteria slowly release the oleic acid at a rate that roughly is proportional to the biomass concentration. Thus the release in a way is regulated by the biomass concentration, but it is also dependent of the rate of enzyme release from the cells. It is contemplated that DATEM in a similar way may function as an oleic acid reservoir. Accordingly a particularly preferred embodiment of the invention is a medium, wherein the DATEM comprise diacetyl tartaric acid esters comprising an oleinate residue.

It should be noted that oleic acid is toxic in high concentrations, since it depolarizes the membrane via a proton transfer from outside the cell into the cell. However the actual effect of oleic acid varies not only with concentration but also with the composition of the fermentation medium. Accordingly, in order to obtain a beneficial effect on the yield it is crucial to select an optimal concentration of DATEM. Thus, in an embodiment of the present invention the medium comprises at least 100 ppm DATEM e. g. at least 500 ppm, such as at least 1000 ppm, e.g. at least 1500 ppm, such as at least 2000 ppm, e. g. at least 2500 ppm or at least 3000 ppm or at least 6000 ppm or even at least 12000 ppm DATEM. In any way the concentration of the DATEM should be optimized according to standard microbiological methods which are well-known to those skilled in the art.

Due to the above discussed antibacterial properties of DATEM one could theoretically believe that an upper concentration limit may be reached which is not conducive to bacterial growth anymore.
As known to the skilled person - in practice it may be difficult to predict the precise ppm value of such a possible maximum/optimal DATEM ppm concentration in the bacterial growth medium.
However, on the other hand it is routine work for the skilled person to simply test this - e.g. by adding increasingly higher amount of DATEM to the bacterial growth medium and then identify optimal DATEM ppm concentration.

Without being limited to theory - it may be that for most industrial practical uses of the present invention an upper concentration limit could be around 12000 ppm DATEM. Accordingly, it may be preferred that the bacterial growth medium comprises from 100 ppm DATEM to 12000 ppm DATEM, or comprises from 500 ppm DATEM to 12000 ppm DATEM or comprises from 1000 ppm DATEM to 6000 ppm DATEM.

Another aspect of the invention is a process for preparing a lactic acid bacterial culture, comprising: culturing at least one strain of lactic acid bacteria in the bacterial growth medium according to the invention; and harvesting the cultured bacteria.

It appears from literature that the growth of most LAB is significantly stimulated by Tween 80 (Johnsson 1995). By analogy it is contemplated that most LAB also are significantly stimulated by DATEM. Therefore an important embodiment of the invention is a process of preparing a lactic acid bacterial culture, wherein the strain of lactic acid bacteria is selected from at least one strain in the group consisting of Lactococus, Lactobacillus, Leuconostoc, Propionibactrium, Bifidobacterium, Oenococcus and Streptococcus. As demonstrated in the examples Tween 80 may advantageously be substituted with DATEM when growing bacteria of the Lactobacillus gasseri and Lactobacillus acidophilus species. Therefore a process, wherein the strain of lactic acid bacteria is selected from at least one strain in the group consisting of Lactobacillus gasseri and Lactobacillus acidophilus strains is a preferred embodiment of the invention. A further preferred embodiment is a process wherein the strain of lactic acid bacteria is a probiotic bacterium. It is noteworthy that the probiotic activity, in general, is associated with specific strains. The LA-5® strain is one example of such a probiotic strain (a comprehensive literature survey is available on request from Chr. Hansen A/S, Hoersholm, Denmark). The Lactobacillus gasseri strain SBT-2055 is also reported to be probiotic. Accordingly the most preferred embodiment is a process, wherein the strain of lactic acid bacteria is selected from at least one strain in the group consisting of Lactobacillus acidophilus strain LA-5 (DSM13241) and Lactobacillus gasseri strain SBT-2055.

It has been reported that improved yields are obtained when culturing a strain of lactic acid bacteria under aeration and in an appropriate nutrient medium, in which at least one porphyrin compound is present or is added (WO 00/05342). Thus one embodiment of the invention is a process wherein the nutrient medium in addition contains at least one porphyrin compound and in particularly a process, wherein the culture is aerated so as to maintain, during the whole duration of the culture, an oxygen content which is at least 5 micromoles per liter of culture medium.

Commercial starter cultures are commonly distributed as frozen cultures. Such concentrated frozen cultures are commercially attractive since such cultures can be inoculated directly into the production container. By using such concentrated frozen cultures the end-user avoids the otherwise obligatory, time-consuming intermediary fermentation step during which the starter cultures are amplified, and the end-user furthermore reduces the risk of contamination drastically. Such concentrated cultures may be referred to as DVS - Direct Vat Set® cultures.

As an alternative to the concentrated frozen cultures concentrated freeze dried DVS® cultures may be prepared. Such cultures have the additional advantage that they can be shipped without refrigeration.

Preferred embodiments of the invention is a process which further comprises freezing or lyophilizing the harvested cultured bacteria, and storing the frozen or lyophilized bacteria.

Importantly, the culture should be packaged in suitable containers, e.g. a special bag (see WO 01/70935) or one of the well-known TetraPack® containers. Accordingly in a further embodiment the process for preparing a lactic acid bacterial culture further comprises packaging the harvested bacteria in a suitable container.

The resulting product of this process is a lactic acid bacterial culture.

As DATEMs in general are considered as safe (GRAS) the bacterial culture obtained by a process according to the present invention may in particular be used for the preparation of a food or feed, food- or feed supplements or for the preparation of pharmaceutical products or medicaments.

The bacterial culture obtained by a process according to the present invention may be given in the form of a fermented food product or in a dosage forms formulated as a tablet (including chewable tablets), a capsule (of either the hard or soft type), a powder, a granulate, a liquid preparation, a suspension, a dried oral supplement, a wet oral supplement, a dry tube feeding formulation or wet tube feeding formulation.

In one embodiment the ingestible material is a fermented food or feed product prepared by use of the bacterial culture obtained by a process according to the present invention. The fermented food or feed product may be further processed. In a number of situations it has been reported that bacteria produces health promoting compounds during fermentation. In such cases it might be advantageous to fractionate and or upconcentrate fractions of the fermented food product. One can even imagine that it in certain situations would be valuable to further process the fermented food product by pasteurization even though the beneficial bacteria are inactivated by such procedure.

By the expression "prebiotic" is referred to a composition or a component of a composition which increases the number of probiotic bacteria in the intestine. Thus, prebiotics refer to any non-viable food component that is specifically fermented in the intestine by indigenous bacteria thought to be of positive value, e.g. lactobacilli. The combined administration of a probiotic strain with one or more prebiotic compounds may enhance the growth of the administered probiotic *in vivo* resulting in a more pronounced health benefit. Therefore one further embodiment of the invention is the use of a composition comprising living probiotic bacteria produced according to the invention in combination with at least one prebiotic. An embodiment wherein the prebiotic is selected from the group: inulin, a transgalacto-oligosaccharide, palantinoseoligosaccharide, soybean oligosaccharide, gentiooligosaccharide, oxylooligomers, nondegradable starch, lactosaccharose; lactulose, lactitol, maltitol, FOS (fructo-oligosaccharides), GOS (galacto-oligosaccharides) and polydextrose is especially preferred.

It is contemplated that the bacterial culture obtained by a process according to the present invention can be used for the preparation of a wide range of ingestible materials such as milk, curd, milk based fermented products, acidified milk, yoghurt, frozen yoghurt, milk powder, milk based powders, milk concentrate, cheese, cheese spreads, dressings beverages, ice-creams, ice-lollies or popsicles, fermented cereal based products, infant formulae and soybean milk.

A further important embodiment of the present invention is the use of the a bacterial culture obtained by a process according to the present invention to prepare a composition for the treatment, prevention of a disease, syndrome or condition, or for improving digestion of nutrients, or for improving the general health status of a human being or a vertebrate animal.

In further embodiments, the culture obtained by a process according to the present invention is used in the production of an animal feed such as silage e.g. grass, cereal material, peas, alfalfa or sugar-beet leaf, where bacterial cultures are inoculated in the feed crop to be ensiled in order to obtain a preservation hereof, or in protein rich animal waste products such as slaughtering offal and fish offal, also with the aims of preserving this offal for animal feeding purposes.

The invention is further illustrated in the following non-limiting examples and tables wherein
Table 1. Shows the yields obtained with a culture of L. gasseri strain SBT2055 cultured in a standard production medium comprising growth stimulating Tween 80, oleic acid or DATEM (Panodan® TR Kosher) compared with the yield obtained with the same medium, but without any of these growth stimulants.
Table 2. Shows the average cell size in a culture of L. gasseri strain SBT2055 cultured in a standard production medium comprising growth stimulating Tween 80, oleic acid or DATEM (Panodan® TR Kosher) and compared with the cell size obtained with the same medium, but without any of these growth stimulants.
Table 3. Shows the yields obtained with a culture of L. acidophilus strain LA-5® cultured in a standard production medium comprising growth stimulating Tween 80 or DATEM (Panodan® TR Kosher) compared with the yield obtained with the same medium, but without any of these growth stimulants.
Table 4. Shows the average cell size in a culture of L. acidophilus strain LA-5® cultured in a standard production medium comprising growth stimulating Tween 80 or DATEM (Panodan® TR Kosher).

### MATERIALS AND METHODS

### Cultures:

The following cultures have been used: Lactobacillus gasseri strain SBT2055, a propriety strain available from Technology and Research Institute, Snow Brand Milk Products Co., Ltd, Kawagoe, Saitama, Japan; and Lactobacillus acidophilus strain LA-5®, deposited on 30-09-2003 according to the Budapest Treaty on the International Recognition of the Deposit of Microorganisms for the Purposes of Patent Procedure with the Deutsche Sammlung von Mikroorganismen und Zellkulturen under accession number DSM13241. LA-5® is commercially available from Chr. Hansen A/S, Denmark.

### Fermentation media and fermentation conditions:

### Medium composition for culture of LAB:

The cultures were cultured in a standard industrial medium comprising hydrolyzed skimmed milk powder 31 g/L, yeast extract 14 g/L, Lactose, acetate and citrate 30 g/L, Mg, MnZn and cystein 0.5 g/L and Tween 80, oleic acid or DATEM as indicated.

The medium was sterilized by UHT-treatment (143°C for 8 sec.). The finished medium had a pH of 6.5.

### Fermentation condition for LA-5:

The fermentation was performed in a 40 L or 15.000 L fermentation tank at 38°C using 1 % (w/w) of the culture mentioned above as inoculum. The anaerobic fermentation was run with nitrogen in the headspace and a headspace pressure of about 0.2 bar. The cultures were allowed to acidify to pH 5.5. The pH was subsequently maintained at 5.5 by controlled addition of 13.4 N NH₄OH.

When no further base consumption was detected, the respective culture was cooled down to about 10°C.

### Fermentation condition for L. gasseri:

The fermentation was performed in a 40 I fermentation tank at 38°C using 1 % (w/w) of the culture mentioned above as inoculum. The anaerobic fermentation was run with nitrogen in the headspace and a headspace pressure of about 0.2 bar. The cultures were allowed to acidify to pH 6.2. The pH was subsequently maintained at 6.2 by controlled addition of 13.4 N NH₄OH.

### Postfermentation treatment of cultures:

Following cooling, the bacteria in fermentation broths were concentrated 10-20 times by centrifugation. CFU measurements were made on the concentrate.

### Additives:

Additives were obtained as indicated: Tween 80 (CRILLET 4, item 20374, Croda Nordica AB), PANODAN® TR Kosher (Danisco A/S, Langebrogade 1, DK-1001 Copenhagen, Denmark), oleic acid (PALMERA A 1813, item 31466, Biesterfeld Spezialchemie GmbH).

### CFU analysis:

CFU analysis was made on concentrated frozen culture CFU analysis. For L. gasseri as described in experimental protocol G-AM-Cul-018. For LA-5 as described in BIRA-QAm-017. Both detailed protocols are available on request from Chr. Hansen A/S, Hoersholm Denmark.

In brief, L. gasseri is diluted and plated on the following medium:

### Identification media (MRS-IM):

| | |
|---|---|
| Tryptone (Oxoid L42) | 10.0 g/L |
| Yeast extract (Oxoid L21) | 5.0 g/L |
| Tween 80 (Merck 822187) | 1.0 g/L |
| K₂HPO₄ (Merck 105104) | 2.6 g/L |
| Sodium acetate (Merck 106267) | 5.0 g/L |
| Di-ammonium-hydrogene-citrate (Merck 101154) | 2.0 g/L |
| Magnesium sulphate, 7H₂O (Merck 105882) | 0.2 g/L |
| Manganese sulphate, H₂O (Merck 105941) | 0.05 g/L |
| Agar | 13.0 g/L |

Plates are incubated for 3 days at 37 °C under anaerobic conditions, and the colonies are counted.

In brief, LA-5 is diluted and plated on the following medium:

### MRS Agar (Difco 288210):

| | |
|---|---|
| Bacto Proteose Peptone No.3 | 10 g/L |
| Bacto Beef Extract | 10 g/L |
| Bacto Yeast Extract | 5 g/L |
| Dextrose | 20 g/L |
| Sorbitan Monooleate Complex | 1 g/L |
| Ammonium Citrate | 2 g/L |
| Sodium acetate | 5 g/L |
| Magnesium sulphate | 0.1 g/L |
| Manganese sulphate | 0.05 g/L |
| Potassium Phosphate Dibasis | 2 g/L |
| Bacto Agar | 15 g/L |

Plates are incubated for 3 days at 37°C under anaerobic conditions, and the colonies are counted.

### Example 1: PANODAN TR Kosher increase the yield of L. gasseri.

In this example the fermentation yield of a L. gasseri culture was measured by the CFU obtained after termination of a fermentation performed as described in the Materials and Methods section.

Four different situations were compared 1) basic medium, 2) basic medium with addition of oleic acid, 3) basic medium with addition of Tween 80 and 4) basic medium with addition of PANODAN TR Kosher.

The results of this experiment are shown in table 1.

**Table 1 Yield of L. gasseri strain SBT2055**

| Addition | amounts g/L | CFU |
|---|---|---|
| none | 0,00 | 6,00x10⁹ |
| Tween 80 | 1.9 | 5,80x10¹⁰ |
| Oleic acid | 0.75 | 9,30x10¹⁰ |
| Panodan TRK | 1.50 | 1,70x10¹¹ |

The results clearly show that addition of a source of fatty acids was important to obtain high CFU numbers.
It has been anticipated for years that oleic acid was the fatty acid that made the high CFU possible whereas other fatty acids may inhibit bacterial growth. Tween 80 comprises an oleic acid residue, and the stimulatory effect of Tween 80 has partly been attributed to its function as an oleic acid-"reservoir".
However, according to the manufacturer's product description - PD 6-11.0EM mat no 076843 Panodan TRK is made from editable sunflower and/or rapeseed oil. In addition to oleic acid sunflower oil also comprise major fractions of palmitic, stearic and linoleic acid. Similarly in addition to oleic acid rapeseed oil also comprise major fractions of palmitic, stearic, linoleic, and linolenic acid.

Thus it was very surprising to the present inventors to learn that a fatty acid source which comprises oleic acid, which originally was considered antibacterial (see US5908862 examples), indeed increased the yield of the fermentation.

Panodan also has a major effect on the morphology of the cells in particular the size of the cells, see Table 2.

**Table 2 Average cell size of L. gasseri strain SBT2055**

| Addition | amounts g/L | Average cell size (10⁻⁶ m) |
|---|---|---|
| none | 0,00 | 5 |
| Tween 80 | 1.9 | 5 |
| Oleic acid | 0.75 | 3 |
| Panodan TRK | 1.50 | 2 |

From these it seems clear that Panodan results in smaller cells. In the case of L. gasseri this is highly advantageous, as such smaller cells are less susceptible to the detrimental effects of centrifugation and further down-stream processing steps, e.g. such as freezing.

### Example 2: PANODAN TR Kosher increase the yield of L. acidophilus.

In this example the fermentation yield of a L. acidophilus LA-5® culture was measured by the CFU obtained after termination of a fermentation performed as described in the Materials and Methods section.

Four different situations were compared 1) basic medium, 2) basic medium with addition of oleic acid, 3) basic medium with addition of Tween 80 and 4) basic medium with addition of PANODAN TR Kosher.

The results of this experiment are shown in table 3.

**Table 3 Yield of L. acidophilus strain LA-5®**

| Addition | amounts g/L | CFU |
|---|---|---|
| Tween 80 | 1.9 | 45 x10⁹ |
| Panodan TRK | 1.5 | 86 x10⁹ |
| Panodan TRK | 3.0 | 100 x10⁹ |

The results clearly show that addition of a source of fatty acids was important to obtain high CFU numbers.

Panodan also has a major effect on the morphology of the cells in particular the size of the cells, see Table 4.

| Table 4 Average cell size of L. acidophilus strain LA-5® | | |
|---|---|---|
| | amounts g/L | Average cell size (10⁻⁶ m) |
| Tween 80 | 1.9 | 5 |
| Panodan TRK | 3.0 | 3 |

From these data it seems clear that Panodan results in smaller cells also in the case of L. acidophilus strain LA-5®. In the case of L. acidophilus strain LA-5® smaller cells are highly advantageous as such smaller cells are less susceptible to the detrimental effects of centrifugation and further down-stream processing steps, e.g. such as freezing.

### REFERENCES

FAO / WHO (2002) Health and Nutritional Properties of Probiotics in Food including Powder Milk with Live Lactic Acid Bacteria. Report of a joint FAO/WHO Expert Consultation. (http://www.mesanders.com/probio_report.pdf)
Mogensen et al. (2002) Bulletin of the IDF No. 377, 10-19
Johnsson et al. APPLIED AND ENVIRONMENTAL MICROBIOLOGY, 61 (12): 4497. (1995)
de Man et al. (1960). J. of Appl. Bact. 23 (I), 130-135
Brubaker et al (1982) Life Sciences Vol 30, p 1965-71)
Nityanand. S, Kapoor. NK (1979) "Effect of chronic oral administration of Tween-80 in Charles Foster Rats". Indian J Med. Res. 1979;69:664-670
Alade (1986) PEDIATRICS Vol. 77, 593-597.
Nieman (1954) Microbiol. Mol. Biol. Rev. 18 (2): 147.
Johnsson et al. (1995) APPLIED AND ENVIRONMENTAL MICROBIOLOGY, 61 (12): 4497
US7217875 B2 "Sunflower seeds and oil having a high stearic acid content".
Alonso (2006) Fuel Processing Technology 87, 97 - 102.
WO05104878A1 "Anti-Microbial Composition".
US5908862A "Water-miscible esters of mono-and diglycerides having antibacterial activity and their use in inhibiting infection".
Food additives in Europe 2000, Nordic Council of Ministers. Copenhagen 2002, ISBN 92-893-0829-X
Material Safety Data Sheet on PANODAN SD-K, Danisco Ingredients, USA, 05.05.99, available from http://www.msds.com.
US2007/178213A1
US5981587

## Claims

1. A process for preparing a lactic acid bacterial culture, comprising:
(a): culturing at least one strain of lactic acid bacteria in a bacterial growth medium, wherein the bacterial growth medium comprises at least 50 ppm of diacetyl tartaric acid esters of mono- and di-glycerides (DATEM); and
(b): harvesting the cultured bacteria.

2. The process of claim 1, wherein said medium comprises at least 500 ppm DATEM.

3. The process of claim 1, wherein said medium comprises at least 1000 ppm DATEM.

4. The process of claim 1, wherein said medium comprises at least 1500 ppm DATEM.

5. The process of claim 1, wherein said medium comprises from 100 ppm DATEM to 12000 ppm DATEM.

6. The process of claim 1, wherein said medium comprises from 500 ppm DATEM to 12000 ppm DATEM.

7. The process of claim 1, wherein said medium comprises from 1000 ppm DATEM to 6000 ppm DATEM.

8. The process according to any of the preceding claims, wherein the strain of lactic acid bacteria is selected from at least one strain in the group consisting of Lactobacillus gasseri and Lactobacillus acidophilus strains.

9. The process according to any of the preceding claims, wherein the strain of lactic acid bacteria is a probiotic strain.

10. The process according to any of the preceding claims, wherein said nutrient medium in addition contains at least one porphyrin compound and the culture is aerated so as to maintain, during the whole duration of the culture, an oxygen content which is at least 5 micromoles per liter of culture medium.

11. The process according to any of the preceding claims, wherein the strain of lactic acid bacteria is selected from at least one strain in the group consisting of Lactobacillus acidophilus strain LA-5 (DSM13241) and Lactobacillus gasseri strain SBT-2055.

12. The process according to any of the preceding claims, and which further comprise a step wherein the culture of lactic acid bacteria is frozen or freeze dried.

13. The process according to any of the preceding claims, and which further comprise a step wherein harvested bacteria are packed in a suitable container.

14. The process according to claims 12 or 13, wherein the obtained bacterial culture is used for the preparation of a food or feed, food- or feed supplements or for the preparation of pharmaceutical products or medicaments.

## Patentansprüche

1. Verfahren zur Herstellung einer Milchsäurebakterienkultur, mit:
(a): Kultivierung mindestens eines Stammes von Milchsäurebakterien in einem Bakterienwachstumsmedium, wobei das Bakterienwachstumsmedium mindestens 50 ppm Diazetylweinsäureester von Mono- und Diglyzeriden (DATEM) umfasst; und
(b): Ernte der gezüchteten Bakterien.

2. Verfahren gemäß Anspruch 1, wobei besagtes Medium mindestens 500 ppm DATEM umfasst.

3. Verfahren gemäß Anspruch 1, wobei besagtes Medium mindestens 1000 ppm DATEM umfasst.

4. Verfahren gemäß Anspruch 1, wobei besagtes Medium mindestens 1500 ppm DATEM umfasst.

5. Verfahren gemäß Anspruch 1, wobei besagtes Medium 100 ppm DATEM bis 12000 ppm DATEM umfasst.

6. Verfahren gemäß Anspruch 1, wobei besagtes Medium 500 ppm DATEM bis 12000 ppm DATEM umfasst.

7. Verfahren gemäß Anspruch 1, wobei besagtes Medium 1000 ppm DATEM bis 6000 ppm DATEM umfasst.

8. Verfahren nach einem beliebigen der vorherigen Patentansprüche, wobei der Stamm von Milchsäurebakterien aus mindestens einem Stamm in der Gruppe bestehend aus Lactobacillus-gasseri- und Lactobacillus-acidophilus-Stämmen ausgewählt ist.

9. Verfahren nach einem beliebigen der vorherigen Patentansprüche, wobei der Stamm von Milchsäurebakterien ein probiotischer Stamm ist.

10. Verfahren nach einem beliebigen der vorherigen Patentansprüche, wobei besagtes Nährstoffmedium zusätzlich mindestens eine Porphyrinverbindung enthält und die Kultur belüftet wird, um während der ganzen Dauer der Kultur einen Sauerstoffgehalt von mindestens 5 Mikromol pro Liter Nährmedium aufrechtzuerhalten.

11. Verfahren nach einem beliebigen der vorherigen Patentansprüche, wobei der Stamm von Milchsäurebakterien aus mindestens einem Stamm in der Gruppe bestehend aus Lactobacillus acidophilus StammLA-5 (DSM13241) und Lactobacillus gasseri Stamm SBT-2055 ausgewählt ist.

12. Verfahren nach einem beliebigen der vorherigen Patentansprüche, welches des weiteren eine Phase umfasst, wobei die Kultur von Milchsäurebakterien gefroren oder gefriergetrocknet wird.

13. Verfahren nach einem beliebigen der vorherigen Patentansprüche, das des weiteren eine Phase umfasst, wobei geerntete Bakterien in einem geeigneten Behälter verpackt werden.

14. Verfahren nach Anspruch 12 oder 13, wobei die erhaltene Bakterienkultur zur Herstellung eines Lebensmittels oder Futters, von Lebensmittel- oder Futterzusätzen oder zur Herstellung von pharmazeutischen Produkten oder Medikamenten benutzt wird.

## Revendications

1. Procédé de préparation d'une culture de bactéries d'acide lactique, comprenant:
(a): la culture d'au moins une souche de bactéries d'acide lactique dans un milieu de croissance bactérienne, où le milieu de croissance bactérienne comprend au moins 50 ppm d'esters d'acide diacétyltartrique de mono- et diglycérides (DATEM); et
(b): la récolte des bactéries cultivées.

2. Procédé selon la revendication 1, où ledit milieu comprend au moins 500 ppm de DATEM.

3. Procédé selon la revendication 1, où ledit milieu comprend au moins 1000 ppm de DATEM.

4. Procédé selon la revendication 1, où ledit milieu comprend au moins 1500 ppm de DATEM.

5. Procédé selon la revendication 1, où ledit milieu comprend de 100 ppm de DATEM à 12000 ppm de DATEM.

6. Procédé selon la revendication 1, où ledit milieu comprend de 500 ppm de DATEM à 12000 ppm de DATEM.

7. Procédé selon la revendication 1, où ledit milieu comprend de 1000 ppm de DATEM à 6000 ppm de DATEM.

8. Procédé selon l'une quelconque des revendications précédentes, où la souche de bactéries d'acide lactique est sélectionnée parmi au moins une souche dans le groupe composé de souches de Lactobacillus gasseri et de Lactobacillus acidophilus.

9. Procédé selon l'une quelconque des revendications précédentes, où la souche de bactéries d'acide lactique est une souche probiotique.

10. Procédé selon l'une quelconque des revendications précédentes, où ledit milieu nutritif contient également au moins un composé porphyrinique et la culture est aérée afin de maintenir, pendant toute la durée de la culture, un contenu d'oxygène d'au moins 5 micromoles par litre de milieu de culture.

11. Procédé selon l'une quelconque des revendications précédentes, où la souche de bactéries d'acide lactique est sélectionnée à partir d'au moins une souche du groupe composé d'une souche de Lactobacillus acidophilus LA-5 (DSM13241) et d'une souche de Lactobacillus gasseri SBT-2055.

12. Procédé selon l'une quelconque des revendications précédentes, comprenant également une phase pendant laquelle la culture de bactéries d'acide lactique est congelée ou lyophilisée.

13. Procédé selon l'une quelconque des revendications précédentes, comprenant également une phase pendant laquelle les bactéries récoltées sont emballées dans un récipient approprié.

14. Procédé selon les revendications 12 ou 13, où la culture bactérienne obtenue est utilisée pour la préparation d'une denrée alimentaire ou aliment, de compléments ou de suppléments alimentaires ou encore pour la préparation de produits pharmaceutiques ou de médicaments.
